Europäisches Patentamt

European Patent Office

Office européen des brevets

(11) Publication number: **0 168 917**
A1

# EUROPEAN PATENT APPLICATION

(21) Application number: 85303218.3

(22) Date of filing: 07.05.85

(51) Int. Cl.⁴: **A 61 N 1/04,** A 61 B 5/04, A 61 L 15/00, A 61 L 25/00, C 08 L 33/26

(30) Priority: 07.05.84 US 607468

(43) Date of publication of application: 22.01.86 Bulletin 86/4

(84) Designated Contracting States: **AT BE CH DE FR GB IT LI LU NL SE**

(71) Applicant: **Ferreira, Lloyd A., 6 Kingsley Court, Princeton Junction New Jersey 08550 (US)**

(72) Inventor: **Ferreira, Lloyd A., 6 Kingsley Court, Princeton Junction New Jersey 08550 (US)**

(74) Representative: **Ellis, John Clifford Holgate et al, MEWBURN ELLIS & CO. 2/3 Cursitor Street, London EC4A 1BQ (GB)**

(54) Conductive material and biomedical electrode.

(57) An electrically conductive material which is cohesive, uniform, microporous, and electrically conductive is described. This material is tacky, is conformable, has low impedance, is essentially non-irritating to the skin and is useful as a conductive material in biomedical electrodes. It comprises a fixed gel of nonionic synthetic polymer and a disaccharide, notably a polyacrylamide-sucrose matrix. It may contain an aqueous salt solution, particularly when used for a biomedical sensing electrode.

EP 0 168 917 A1

ACTORUM AG

## CONDUCTIVE MATERIAL AND BIOMEDICAL ELECTRODE

The present invention relates to a material, useful in detection and stimulating electrodes, as an electrical conductor. This material is also useful as a carrier for the dispensing of medicaments. Furthermore, it can be used in the treatment of burns and wounds, and for the protection and treatment of healing limbs after amputation, for example.

The present invention also relates to a biomedical electrode containing the electrically conductive material of the present invention.

There are a number of biomedical electrodes known in the art. In general, these electrodes are comprised of a metallic electrode plate which allows for the attachment of a lead wire which connects to an electromedical apparatus. Contact between the electrode and the skin is made via a conductive material which is coated on the surface of the electrode (an essentially "dry" electrode) or by means of gels, pastes or creams which are either applied to the skin surface directly or in contact with the skin via a mesh or sponge which is impregnated with a conductive material. Another means of providing for electrical conductivity in electrodes is to provide a carrier (usually an adhesive) with conductive particles, such as metals, distributed therein.

Another means of providing conductivity between a metallic electrode and the skin is to use an adhesive which has had dispersed within it a salt in an attempt to increase the conductivity of the electrode.

Examples of the use of pastes, creams or gels can be found in the prior art.

Examples of the use of a porous pad impregnated with a conductive material can also be found in the prior art.

These conductive pastes, creams or gels when used in prior art medical electrodes are somewhat unpleasant to use in that they are sloppy, and are often irritating to the skin, primarily because of their ionic nature, especially when the skin is cleaned and abraded prior to application of the electrode. A further disadvantage is the residue which is left on the skin after removal of the electrode. This requires the additional step of cleaning up after the use of these electrodes is completed.

While somewhat more convenient than the use of the pastes, creams or gels, porous pads impregnated with conductive materials nevertheless require expensive and elaborate packaging in order to prevent the materials from drying out. Furthermore, the gels have a tendency to dry out when in use, which causes an electrical variation in impedance with a subsequent effect on the quality of the signal when these materials are used.

The use of adhesives impregnated with conductive materials can be found in the prior art. An example of an adhesive with a salt dispersed within it can also be found in the prior art. Both of these groups of materials suffer from the fact that the use of these materials can cause skin irritation, can result in "hot spots" which would effect the signal quality, and the possibility of an increase in overall cost of the electrode is quite real.

Examples of "dry" electrodes can be found in the prior art.

The primary disadvantages of using these electrodes is lack of adequate conformability with the body surface, with certain electrodes requiring activation by use of an aqueous solution. However, these electrodes generally have a relatively high impedance, thus requiring larger amounts of electricity to effectively utilize them.

In contrast, however, the present invention overcomes these problems.

The present invention provides a cohesive, uniform, microporous, electrically conductive material which is tacky, conformable, has low impedance, is essentially non-irritating to the skin, and contains no particles of metal, said material comprising a fixed gel of non-ionic synthetic polymer and a disaccaride.

A test piece of the material having faces one square inch in area and 1/16 inch thick preferably has an impedance of less than 20 ohms at a frequency of 1-500 KHertz as measured across electrodes covering the said faces.

The conductive material of the present invention is a fixed gel with an additive or ingredient to provide properties of tackiness and flexibility and conformity such that the gel is particularly suitable for employment on various areas of the body. A particularly suitable gel is one comprising polyacrylamide which is insoluble and sucrose, which when used as a biomedical sensing electrode, contains an aqueous solution of salts.

Since the material of the present invention is a fixed gel, it has the following properties and advantages: it is not runny as in the instance of the use of pastes, gels and creams. It is of uniform consistency and therefore does not have "hot spots". It also allows for much greater accuracy when used in electrodes since it has a very low impedance and thus requires much less current to operate in either the sensing or stimulating mode. The material of the present invention does not require the use of a carrier since it is a self-contained material, i.e., a fixed gel, and can have up to 95% liquid contained therein.

The material of the present invention is flexible and can therefore conform to the skin surface on all parts of the body.

When used as an electrode conductive material, it is tacky and thus requires little, if any, additional adhesive to retain it on the skin's surface. The material of the present invention is non-irritating to the skin, even when the skin has been abraded.

The material of the present invention is cool to the touch, possibly due to evaporation of a microscopic layer of moisture from the surface of the self-contained material. This feature, coupled with the fact that a low amount of current can be used in the operation of an electrode using this material, effectively eliminates the feeling of irritation from the skin.

Furthermore, because of the advantage of lack of irritability, electrodes which must be worn for long periods of time, when utilizing the conductive material of the present invention, are much more comfortable to wear than those employing prior art materials. Testing has shown that no noticeable irritation resulted from the use of this material worn by patients over many days.

The material of the present invention, being a fixed gel, is believed to behave as if it were a layer of ionized water between the electrode and the skin. The water content of the conductive material of the present invention can be as high as 95%, with the remaining 5% comprised of the polyacrylamide-sucrose matrix, and, optionally, electrolyte, a fungicide, antiseptic, scent, and colouring agent.

The material of the present invention, in general, has a low rate of shrinking and/or swelling and can be easily brought to equilibrium by adding or removing liquid, respectively. Consequently, no expensive packaging or special procedures are required. In fact, at ambient conditions, the material never completely dries out, retaining at least 10% moisture even after exposure to air for long periods of time.

The pH of the material of the present invention can be adjusted to that of the skin. It can also be made chloride ion-free and these factors minimize the irritability to the skin.

The fact that the present invention has a low impedance requires the use of smaller amounts of current in the use of this material in a detection or stimulating electrode. The impedance of electrodes made from the present invention is in the range of 1 to 20 ohms at 1-500 KHertz. In comparison, prior art electrodes have impedances ranging from 50-2000 ohms at 1-500 KHertz. The higher impedances of prior art materials require more current, thus generating heat to the electrodes and increasing the probability of irritation of the skin of the patient.

The material of the present invention has other uses. Amongst them are burn treatment, the dispensing of medication, cushioning and protecting healing wounds, transmission and absorption of gases, absorption of material from oozing wounds, and the use of the material in ultrasonics for sound and vibration treatments of patients.

Because of the special structure of the material of the present invention, i.e., a fixed gel matrix, it acts as a very efficient transport system of electrical current, i.e., from D.C. to 500 KHz, medicaments, fluids and gases for patients requiring the treatments noted above.

The pore size can be varied so that materials having molecular weights of from $3 \times 10^6$ to $1 \times 10^5$ Daltons can be passed through the matrix of the present invention. Furthermore, the material of the present invention can be adjusted so that equilibrium diffusion times can be from between 1 hour to 4 hours depending on the rate of delivery of the medication required.

The material of the present invention can be made in very uniform thickness with a variance of less than $\pm$ 0.01 mm. Alternatively, the material of the present invention can be formed into blocks and shaped and molded to act as a cushion and support for those patients requiring this kind of treatment, such as amputees and burn patients.

The tackiness of the material of the present invention is adjustable depending on the use of the material. Removal of the material from patients wearing or utilizing these devices is simple and leaves essentially no residue.

The degree of tackiness is in direct relation to the amount of sucrose present in the composition of the present invention. The greater the amount of sucrose, the greater the tackiness. Furthermore, the increase in the amount of sucrose is directly related to the confirmability to the patient's skin, as well.

In one aspect of the invention, the polyacrylamide-sucrose matrix material of the present invention, after polymerization, is poured into trays to a given thickness and, after setting, a clear sheet of soft and elastic material is obtained which can be cut into pre-determined shapes quite easily. For instance, it can be pre-cut to size (e.g. punched out) for placement into a biomedical electrode shell using simple procedures since, as a result of its tackiness, it will stick to the underside of the electrode plate quite easily. The sheet material can also be easily adapted for use at the site of the patient, because of its ease of manipulation.

Blocks of the material can be prepared by pouring the polymerized material into molds of a predetermined size and shape. This material can be easily adapted for covering and cushioning wounds and for the transport of medication, ultrasonics, electrical current, heat or cold to and, where applicable, from the required area of the patient.

GENERALIZED EXAMPLE

Acrylamide, in the range of from 2 to 30% by weight, sucrose, in the range of from 1 to 50% by weight, 0.025-0.05% by weight of bis-acrylamide (a cross-linking agent), 0.01-0.03% by weight of ammonium persulfate (an initiator) and 0.01-0.05% by weight of TEMED (tetramethyl ethylenediamine, an initiator) in an aqueous solution, is polymerized. The polymerization can take place at room temperature without de-aeration.

The polymerization is essentially completed in about 30 minutes. Increasing the temperature speeds the polymerization reaction. The reaction vessel and the starting liquids can be sparged with an inert gas such as nitrogen to minimize inhibition of reaction by oxidation.

Ionic salts can be included in the polymerization reaction vessel. Examples of these ions are sodium, potassium, acetate, sulfate, phosphate and chloride.

The polymerized material is poured into trays, or into molds of predetermined size. The resulting material, after setting, can be pre-cut into desired shapes and packaged, or can be fitted into electrode shells and packaged.

As noted above, the greater the amount of sucrose, the more tacky will be the resulting gel. Furthermore, the lower the amount of cross-linking agent, the greater the elasticity and thus, the moldability and conformability of the resulting gel material.

Any residual unreacted materials, after the polymerization, can easily be removed by soaking the gel material in solutions containing purified water and proper concentrations of sucrose, although the polymerization can be run to essentially completion in practice.

A.    Stimulator Electrode Evaluation Tests

The purpose of these tests is to measure and compare current output into the human body of various known electrodes against the present invention.

0168917

The tests were run with the following constants:

1) the surface area of each of the electrodes tested was 2.25 sq. inches.

2) the electrodes were placed on the dorsal web space (the fleshy area between the thumb and forefinger) which had been cleaned to remove any skin oils. The leads were connected to a constant voltage pulse generator (12 volts, 150μ sec. 82 p.p.s- (pulse per second) with a 10 ohm resistor in the ground lead, across which was connected a tracing oscilloscope. All equipment was temperature stabilized (ambient temperature) and calibrated.

The electrodes were allowed to stabilize for 20 seconds after placement and measurements were then taken.

### RESULTS

Testing of an electrode using the gel material of the present invention as described above with a thickness of 1/16 inch, against prior art electrodes, gave the results as noted in Table I below:

12.

## Table 1

Current Output, mAmps

| | | |
|---|---|---|
| 1. | U.S. Patent No. 4,066,078 | 29 |
| 2. | U.S. Patent No. 3,994,302 | 27 |
| 3. | Lectic Karaya Electrode | 27 |
| 4. | U.S. Patent No. 4,273,135 | 19 |
| 5. | U.S. Patent No. 3,998,215 | 17 |
| 6. | EMPI S.U.E. Karaya | 11 |
| 7. | Polyacrylamide Polymerized with 33% Glycerin | 40 |
| 8. | Present Invention (Acrylamide Polymerized with 33% Sucrose) | 42 |

The results show that the electrode of the present invention is at least 30% more efficient than most of the prior art electrodes tested. This fact, coupled with the added features noted above, show the surprising and unobvious advantages of the present invention over the prior art.

The electrode material of Item 7, above, while quite efficient, lacks other properties of the present invention, such as good compression and skin adhesion characteristics, as shown below.

B.   Compression and Skin Adhesion Tests.

The gel material of the present invention shows excellent recovery in comparative compression tests and in skin adhesion.

The requirement for good compression recovery is necessary in an electrode since poor compression characteristics could result in "hot spots" when the electrode is placed against the skin because of movement of the conductive material by compression. This could result in uneven distribution of the current input in the stimulating mode, or in uneven and spurious signals in the sensing mode.

The requirement for good skin adhesion in an electrode is necessary to minimize movement in order to allow for maintaining localization of current input in the stimulating mode and for cleaner signals in the sensing mode. An external adhesive source is thus essentially eliminated, minimizing the incidence of skin irritation.

The compression tests (see Table II) were run on prior art materils noted below and on a 2 centimeter diameter disc of the present invention. Measurements of deflection and recovery after applying weights are noted below.

The skin adhesion tests were performed using 1 x 1 and 1 x 2 inch size materials when a load was attached thereto at right angles to the skin until failure.

14.

## Table II

| Material | Compression | | | Skin-Right Angle Shear | | |
|---|---|---|---|---|---|---|
| | 50gr | 100gr | 150gr | | 2 sq inch | per sq inch |
| Present Invention Acrylamide sucrose | 0.05mm recovery; 100% | 0.10mm | 0.15mm | not wet | 140g | 70g |
| Karaya Gum Electrode | 0.05mm recovery; 5% | 0.05mm | 0.05mm | not wet wet | 0 0 | 0 0 |
| "Dry Electrode" U.S. Pat. No. 4,273,135 | recovery; less than 0.01% | | | not wet wet | 0 10g | 0 5g |
| Acrylamide-no sucrose | recovery; less than 0.01% | | | not wet wet | 0 10g | 0 5g |
| Acrylamide glycerine 33% | 0.10mm recovery; 20% | 0.20mm | 0.30mm | not wet | 25g | 13g |

The compression tests show 100% recovery for the gel material of the present invention, while the closest prior art material shows only a 20% recovery, a factor of 5.

The skin adhesion tests show an adhesion which is at least 5 times greater than the closest prior art material.

Drug delivery systems using the fixed gel material of the present invention can conveniently be prepared by introducing the respective drugs by, for example, diffusion techniques into the fixed gel, or they may be incorporated into the monomer mixture before preparation of the gel. The molecular weights of these materials can range up to $10^6$ Daltons.

Fungicides, antibiotics, scents, FD and C colouring agents can be introduced by conventional techniques.

Tests on rabbits of the conductive material of the present invention were performed both on normal rabbit skin or abraded rabbit skin over a 24 hour period in the first series of tests and over a second series of tests conducted over a 72 hour period.

The biomedical electrode of the present invention utilizes conventional parts with the exception of the use of the electrically conductive material of the present invention in place of that which is known in the art.

The electrode comprises an electrode plate designed to collect the electrical potential. For example, it may have an upper surface and a lower surface, the upper surface having means for electrically connecting an electrode plate to a lead wire, said lead wire subsequently connected to a medical apparatus. The conductive material of the present invention is attached to the lower surface of the electrode plate and the electrically conductive material of the present invention is brought in contact with the skin of the patient, when the electrode is to be used. The electrode utilizing the fixed gel conductive material of the present invention has an impedance of 1-20 ohms or less at a frequency of 1-500 KHertz. The material used for the electrode plate can be any of the conventional metals or can be an electrically conductive plastic. Preferably, for use as a disposable electrode, stainless steel can be used.

The present invention provides for a cohesive, uniform, microporous, electrically conductive material which is tacky, conformable, has low impedance, is essentially non-irritating to the skin, and is non-metallic and is comprised of a non-ionic synthetic polymer which will provide an electrode with an impedance of 1-20 ohms or less at a frequency of 1-500 KHertz. This material is generally a polymer which is a polyacrylamide-sucrose matrix having a pore size sufficient to allow the passage of materials and solution in the molecular weight range of $3 \times 10^6$ to $1 \times 10^5$ Daltons.

The sucrose content of the polyacylamide matrix ranges from 2 to 50% by weight. The material also may contain an electrolyte solution comprising various ions, as noted above.

The gel material of the present invention may also contain a fungicide and/or antiseptic and may also contain a scent and/or a pharmaceutically acceptable colouring agent.

The present invention also includes the use of the electrically conductive material of the present invention in a biomedical electrode.

Said biomedical electrode is comprised of an electrode plate having an upper and lower surface, said upper surface having means attached thereto for connecting said electrode plate to a lead wire and said lower surface having attached thereto an upper surface of an electrically conductive material of the present invention, said electrically conductive material contacting the skin via a lower surface thereof when the electrode is in use.

18. 0168917

CLAIMS:

1.   A cohesive, uniform, microporous, electrically conductive material which is tacky, conformable, has a low impedance, is essentially non-irritating to the skin, and contains no particles of metal, said material comprising a fixed gel of non-ionic synthetic polymer and a disaccharide.

2.   An electrically conductive material according to claim 1, wherein said gel is a polyacrylamide-sucrose matrix having a pore size sufficient to allow the passage of materials in solution in the molecular weight range of up to $3 \times 10^6$ Daltons

3.   An electricaly conductive material according to claim 1 or claim 2, wherein said gel is a polymer having a polyacrylamide-sucrose matrix which contains sucrose in the amount from 2% to 50% by weight.

4.   An electrically conductive material ccording to claim 1, 2 or 3, which contains an electrolyte impregnated therein comprising sodium ions, potassium ions, acetate ions, sulfate ions, chloride ions and/or phosphate ions.

5.   An electrically conductive material according to any one of the previous claims which additionally contains a fungicide and/or antiseptic.

6.   An electrically conductive material according to any

19. 0168917

one of the previous claims which additionally contains a scent and/or pharmaceutically acceptable colouring agent.

7. A biomedical electrode comprising an electrode plate for gathering the electrical potential, having means attached thereto for connecting said electrode plate to a lead wire, characterized in having attached thereto an upper surface of the electrically conductive material of any one of the previous claims, said electrically conductive material contacting the skin via a lower surface thereof when the electrode is in use.

8. A biomedical electrode according to claim 7 with an impedance of 1-20 ohms or less at a frequency of 1-500 KHertz.

9. An electrically conductive material according to any one of claims 1 to 6 having an impedance of less than 20 ohms at a frequency of 1-500 KHertz for a test piece one inch square and 1/16 inch thick.

European Patent Office

**EUROPEAN SEARCH REPORT**

## DOCUMENTS CONSIDERED TO BE RELEVANT

EP 85303218.3

| Category | Citation of document with indication, where appropriate, of relevant passages | Relevant to claim | CLASSIFICATION OF THE APPLICATION (Int. Cl 4) |
|---|---|---|---|
| X | <u>DD - A - 145 062</u> (MAX-PLANCK-GE-SELLSCHAFT) <br> * Abstract; claims 1,4-9 * <br> -- | 1-6 | A 61 N 1/04 <br> A 61 B 5/04 <br> A 61 L 15/00 <br> A 61 L 25/00 <br> C 08 L 33/26 |
| A | <u>GB - A - 2 114 578</u> (KIEVSKY) <br> * Abstract; page 4, line 62 - page 5, line 9; page 6, lines 8-10 * <br> -- | 1,4 | |
| A | <u>GB - A - 1 594 389</u> (MAX-PLANCK-GE-SELLSCHAFT) <br> * Claims 1-8 * <br> -- | 1-6 | |
| A | <u>US - A - 4 306 551</u> (HYMES) <br> * Abstract; claims 1,2 * <br> -- | 1 | |
| A | <u>AT - B - 367 300</u> (SEIDERMAN) <br> * Claims 1-3 * <br> -- | 1-7 | TECHNICAL FIELDS SEARCHED (Int. Cl 4) <br> A 61 N <br> A 61 B <br> A 61 L <br> C 08 L |
| A | <u>AT - B - 365 932</u> (RHODES) <br> * Page 3, lines 43-52; page 4, lines 12-17, 38-42; claim * <br> -- | 1,4-7 | |
| A | <u>EP - A1 - 0 085 327</u> (MEDTRONIC) <br> * Abstract; claims 1,5-7,9,11 * <br> -- | 1-4,7 | |
| A | <u>EP - A2 - 0 004 514</u> (GUILLOT) <br> * Abstract; claims 1,4 * <br> -- | 1,6,7 | |

The present search report has been drawn up for all claims

| Place of search | Date of completion of the search | Examiner |
|---|---|---|
| VIENNA | 30-09-1985 | NEGWER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

## European Patent Office

### EUROPEAN SEARCH REPORT

## DOCUMENTS CONSIDERED TO BE RELEVANT

EP 85303218.3

| Category | Citation of document with indication, where appropriate, of relevant passages | Relevant to claim | CLASSIFICATION OF THE APPLICATION (Int. Cl 4) |
|---|---|---|---|
| A | WO - A1 - 81/785 (MINNESOTA MINING AND MANUFACTURING) <br> * Abstract; claims 1-3,5-8 * | 1,4,7 | |
| A | CA - A - 1 144 606 (GRAPHIC CON-TROLS CANADA) <br> * Claims 1,4 * | 1,4,7 | |
| A | DE - A1 - 2 521 697 (MEDTRONIC) <br> * Claims 1,6 * | 1,7 | |
| A | US - A - 4 125 110 (HYMES) <br> * Abstract; claims 1-3,8,11 * | 1,4,7 | |
| A | US - A - 4 391 278 (CAHALAN) <br> * Abstract; claims 1,4 * | 1,7 | TECHNICAL FIELDS SEARCHED (Int. Cl 4) |

The present search report has been drawn up for all claims

| Place of search | Date of completion of the search | Examiner |
|---|---|---|
| VIENNA | 30-09-1985 | NEGWER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82